# EUROPEAN PATENT SPECIFICATION

(11) **EP 2 661 474 B1**
(45) Date of publication and mention of the grant of the patent: **24.06.2015**
(21) Application number: 11807764.3
(22) Date of filing: 13.12.2011
(51) Int. Cl.: C09K 11/74, C09K 11/77, H05B 33/14, H05B 33/18, C09K 11/08, A61B 18/18, H01J 61/40

(54) **UV-EMITTING PHOSPHORS**
UV-EMITTIERENDE LEUCHTSTOFFE
LUMINOPHORES ÉMETTANT DES RAYONNEMENTS UV

(30) Priority: 04.01.2011 EP 11150063
(43) Date of publication of application: 13.11.2013
(73) Proprietor: Koninklijke Philips N.V., 5656 AE Eindhoven (NL); Philips Intellectual Property & Standards GmbH, 20099 Hamburg (DE)
(72) Inventor: GREUEL, Georg, NL-5656 AE Eindhoven (NL); PLEWA, Julian, NL-5656 AE Eindhoven (NL); BETTENTRUP, Helga, NL-5656 AE Eindhoven (NL); JUESTEL, Thomas, NL-5656 AE Eindhoven (NL)
(74) Representative: van Eeuwijk, Alexander Henricus Waltherus
(86) International application number: PCT/IB2011/055626
(87) International publication number: WO 2012/093298

(56) References cited:
- WO-A2-2006/109238
- SUSHIL K. MISRA ET AL: "EPR of the Kramers ions Er3+, Nd3+, Yb3+ and Ce3+ in Y(NO3)3.6H2O and Y2(SO4)3.8H2O single crystals:", PHYSICA B: CONDENSED MATTER, vol. 253, no. 1-2, 1 October 1998 (1998-10-01), pages 111-122, XP55021723, ISSN: 0921-4526, DOI: 10.1016/S0921-4526(98)00373-1
- IVAN ROUSE ET AL: "Microwave resonance studies on Tm^{3+}(4f^{12}) diluted into single-crystal monoclinic YCl_{3}.6H_{2}O and Y_{2}(SO_{4})_{3}.8H_{2}O", PHYSICAL REVIEW B, vol. 13, no. 9, 1 May 1976 (1976-05-01), pages 3764-3773, XP55021757, ISSN: 0556-2805, DOI: 10.1103/PhysRevB.13.3764
- SUSHIL MISRA ET AL: "EPR study of Gd3+-doped RbR(SO4)2.4H2O (R=Pr, Nd, Sm, Eu) single crystals:? Phase transitions and spin-Hamiltonian parameters", PHYSICAL REVIEW B, vol. 56, no. 5, 1 August 1997 (1997-08-01) , pages 2391-2398, XP55021739, ISSN: 0163-1829, DOI: 10.1103/PhysRevB.56.2391

## Description

### FIELD OF THE INVENTION

The present invention relates to the field of UV emitting luminescent compounds e.g. for use in UV lamps.

### BACKGROUND OF THE INVENTION

Fluorescent lamps which comprise an UV emitting phosphor are widely used for many applications, including disinfection and purification, skin tanning, medical treatments of the skin, polymer hardening, and semiconductor wafer processing.

Conventionally, UV radiation sources are based on low- or medium-pressure mercury (Hg) discharge (also referred to as mercury-vapor lamps). The emission line spectrum of a low-pressure Hg discharge is dominated by the 185 nm and 245 nm lines. Increasing the pressure results in line broadening, increasing emission in the visible spectrum at the cost of UV emission. Hence, medium-pressure Hg discharge are less efficient as UV emitters. The efficiency of Hg discharge lamps is strongly dependent on temperature due to the change in Hg vapor pressure. Moreover, the spectrum changes with lamp drive conditions and temperature, which is undesirable.

In recent years, dielectric barrier excimer discharge has drawn attention as an alternative discharge source for UV emitting discharge lamps. An excimer discharge lamp is a discharge lamp in which at least one component of the discharge-maintaining gas filling forms an excimer during operation of the lamp. Xenon (Xe) excimer discharge emits light mainly of 172 nm, and dielectric barrier driven quartz lamp comprising Xe as a filling gas shows a wall plug efficiency of more than 50 %. Besides Xe as the excimer-forming gas filling component there are other well known excimer-forming filling components like Ne.

Xe excimer discharge lamps using a fluorescent compound for conversion of discharge emission into visible light or into UV light have been described, e.g. in US 2008/02588601. However, presently known UV emitting wavelength converting materials, also referred to as UV-emitting luminescent materials or UV-emitting phosphors, suffer from a number of drawbacks, such as undesirably low conversion efficiency, low photochemical and/or chemical stability, Lewis alkalinity, undesirable chemical interaction with the discharge resulting in degradation of the UV-emitting luminescent material, and undesirably low disinfection efficiency due to spectral mismatch with the germicidal action curve. For example, the chemical instability of the luminescent materials of US 2008/02588601 in the presence of the excimer discharge may require a protective coating e.g. of alumina, which reduces the conversion efficiency.

Hence, there remains a need in the art for improved UV emitting lamps and materials used therein.

In the non-patent literature D1, a material is disclosed comprising Y₂(SO₄)₃ *8H₂O doped with 1% of Pr³⁺ or 1% of Nd³⁺ (table 2; page 112, left column, last paragraph). Said material has a monoclinic crystal structure. The wavelength converting material of the present invention is distinguished from this material by the absence of crystal water.

D1: SUSHIL K. MISRA ET AL: "EPR of the Kramers ions Er3+, Nd3+, Yb3+ and Ce3+ in Y(NO3)3.6H2O and Y2(SO4)3.8H2O single crystals:", PHYSICA B: CONDENSED MATTER, vol. 253, no. 1-2, 1 October 1998 (1998-10-01), pages 111-122, ISSN: 0921-4526, DOI: 10.1016/S0921-4526(98)00373-1

### SUMMARY OF THE INVENTION

It is an object of the present invention to at least partly overcome the drawbacks of the prior art, and to provide a UV emitting light source which has improved efficiency and/or which is particularly useful for UV sterilization.

According to a first aspect of the invention, this and other objects are achieved by a wavelength converting material comprising a compound of the formula:

(Y_{1-w-x-y-z}Sc_{w}LaₓGd_{y}Lu_{z})₂₋ₐ(SO₄)₃:Meₐ

wherein Me represents a trivalent cation or a mixture of trivalent cations capable of emitting UV-C radiation, and wherein each of w, x, y and z is in the range of from 0.0 to 1.0 and w+x+y+z ≤ 1.0, and wherein 0.0005 ≤ a ≤ 0.2. The wavelength converting material provides intense and efficient conversion of UV light of the wavelength range of 100 to 200 nm into light of the wavelength range of 200 to 300 nm. The wavelength converting material thus has high quantum efficiency. Furthermore, the UV emission from the present wavelength converting material has a high degree of overlap with the germicidal action curve (GAC), for example an integral overlap of at least 70 %. The present wavelength converting material is also temperature stable and thus tolerates the high operating temperature of an excimer lamp. Additionally, the rare earth metal sulphates are easy to produce.

In embodiments of the invention, Me is at least one of trivalent praseodymium ions (Pr³⁺), neodymium ions (Nd³⁺) and bismuth ions (Bi³⁺). The 4f-5d transitions of Pr³⁺ and Nd³⁺ provide fast luminescence, having a large absorption cross-section (high probability of an absorption process), and result in efficient wavelength converting materials. This is also true for the 6s-6p transition of Bi³⁺.

In embodiments of the invention, a is in the range of 0.002 to 0.1, and typically is in the range of 0.01 ot 0.04 (corresponding to an Me content of 0.5 to 2 at.%). Such contents of the dopant (Me) provides particularly good absorption and avoids or reduces concentration quenching.

In embodiments of the invention, Me comprises at least one of Pr³⁺, Nd³⁺ and Bi³⁺ and additionally at least one further trivalent cation. By using such a co-dopant, the emission spectrum of the wavelength converting material can be adjusted to even better suit the intended application.

In another aspect, the present invention relates to a wavelength converting screen or a wavelength converting coating, comprising a wavelength converting material as defined above. Such a screen or coating is typically used in an excimer discharge lamp, in which it is arranged to receive UV light resulting from the discharge.

Hence, in another aspect, the invention provides an illumination device comprising a source of UV light to be converted, i.e. a source of unconverted light, and a wavelength converting material or a wavelength converting screen or coating as described above, for converting the UV light from said source. Typically, the illumination device may be a discharge lamp comprising a discharge vessel containing a gas comprising one or more of Ar, Kr, Xe, F₂, Cl₂, Br₂, and I₂, and means for creating an electrical discharge, and wherein at least part of a wall of the discharge vessel is provided with a wavelength converting material as described above, e.g. in the form of a coating.

In embodiments of the invention, the illumination device may be or form part of a medical device or a cosmetic treatment device.

In another aspect, the invention provides a cosmetic treatment device comprising a wavelength converting composition as defined above, a wavelength converting screen or coating as defined above, and/or an illumination device as defined above.

In a further aspect, the invention provides a medical device comprising a wavelength converting composition as defined above, a wavelength converting screen or coating as defined above, and/or an illumination device as defined above. For example, the medical device may be a phototherapy device.

The illumination device described above may form part of a system for UV illumination comprising e.g. optical components, such as lenses, waveguides etc, control circuitry and devices, cooling arrangements, mechanical support structures, etc. Such a system may be a system for sterilization, disinfection and/or purification by germicidal UV illumination. Alternatively, a system comprising said illumination device may be a medical device system. Alternatively, said system may be a chemical reactor, or a photoetching equipment.

In another aspect, the present invention relates to the use of a wavelength converting composition as defined above for sterilization, disinfection or purification.

In yet another aspect, the invention relates to the use of a wavelength converting composition as defined above in a cosmetic treatment method, typically tanning.

In a further aspect, the invention provides a method of producing a wavelength converting composition as defined above, comprising: reacting an oxide of Y, Lu, Sc La or Gd, with a sulphate or oxide of said trivalent cation in a sulphuric acid-containing medium; and removing said medium. These steps are typically followed by a step of disintegrating, e.g. milling, the reaction product, and then by annealing the disintegrated reaction product, to obtain a crystal lattice of rare earth metal orthosulphate containing a dopant dispersed therein.

It is noted that the invention relates to all possible combinations of features recited in the claims.

### BRIEF DESCRIPTION OF THE DRAWINGS

This and other aspects of the present invention will now be described in more detail, with reference to the appended drawings showing embodiment(s) of the invention.
- Fig. 1: shows the emission spectrum of a Pr³⁺ doped yttrium orthosulphates at various dopant concentrations.
- Fig. 2: shows the emission spectrum of a Pr³⁺ doped yttrium orthosulphate and the germicidal action curve.
- Fig. 3: is a schematic cross-sectional view of a UV emitting discharge lamp according to embodiments of the invention.
- Fig. 4a: shows the XRD pattern of a wavelength converting compound according to an embodiment of the invention (Y₂(SO₄)₃:Pr).
- Fig. 4b: shows the reflection, excitation and emission spectra of a wavelength converting compound according to an embodiment of the invention (Y₂(SO₄)₃:Pr).
- Fig. 5a-b: show respectively the XRD pattern and the reflection (unbroken line), excitation (broken line) and emission (dotted line) spectra of another wavelength converting compound according to an embodiment of the invention (Lu₂(SO₄)₃:Pr).
- Fig. 6a-b: show respectively the XRD pattern and the reflection (unbroken line), excitation (broken line) and emission (dotted line) spectra of another wavelength converting compound according to an embodiment of the invention (La₂(SO₄)₃:Pr).
- Fig. 7a-b: show respectively the XRD pattern and the reflection, excitation and emission spectra of another wavelength converting compound according to an embodiment of the invention (Y₂(SO₄)₃:Nd).
- Fig. 8 a-c: show respectively the XRD pattern and the reflection (Fig. b) and emission (Fig. 8c) spectra of another wavelength converting compound according to the embodiment of the invention (Lu₂(SO₄)₃:Bi) described in Example V.
- Fig. 9a-b: show respectively the XRD pattern and the reflection, excitation and emission spectra of another wavelength converting compound according to an embodiment of the invention (Lu₂(SO₄)₃:Nd).

### DETAILED DESCRIPTION

The present inventors have found that certain rare earth metal oxide sulfates doped with Pr³⁺, Nd³⁺ or Bi³⁺ have excellent properties for use as VUV to UV-C converting compounds e.g. for use in excimer discharge lamps.

The wavelength converting compounds according to the invention have the general formula Ln₂(SO₄)₃:Me, or more specifically Ln₂₋ₐ(SO₄)₃:Meₐ (which may also be written Ln₂₋ₐMeₐ(SO₄)₃), in which Ln is one or more of yttrium (Y), scandium (Sc), lanthanum (La), gadolinium (Ga) and lutetium (Lu), and Me is a trivalent cation, a being in the range from 0.0005 to 0.2. In particular, the wavelength converting compound has the formula

(Y_{1-w-x-y-z}Sc_{w}LaₓGd_{y}Lu_{z})₂₋ₐ(SO₄)₃:Meₐ,

wherein each of w, x, y and z is in the range from 0.0 to 1.0 and w+x+y+z ≤ 1.0, and wherein Me and a respectively are as defined above.

In embodiments of the invention, a may be in the range from 0.001 to 0.1, typically from 0.002 to 0.1, or from 0.01 to 0.04.

In the wavelength converting compounds of the present invention, the sulfate of Sc, Y, La, Gd and/or Lu provides a host lattice which is activated by small amounts of Me as an activator, also referred to as a dopant. Me represents a trivalent cation, typically ions of bismuth (Bi³⁺), praseodymium (Pr³⁺) or neodymium (Nd³⁺). The activator Me is capable of emitting UV light in the range of 200-300 nm.

As used herein, a wavelength converting compound refers to a compound which is capable of absorbing electromagnetic radiation of a particular wavelength or wavelength range and of emitting electromagnetic radiation of a different wavelength or wavelength range, typically of a longer wavelength.

As used herein, a wavelength converting material refers to a material having the same capability of absorption and emission as a wavelength converting compound. A wavelength converting material may be composed of a single type of wavelength converting compound or of a mixture of different types of wavelength converting compounds.

As used herein, the term "activator" or "dopant" refers to an impurity present in a host lattice, in particular trivalent ions, which is capable of emitting UV radiation upon excitation.

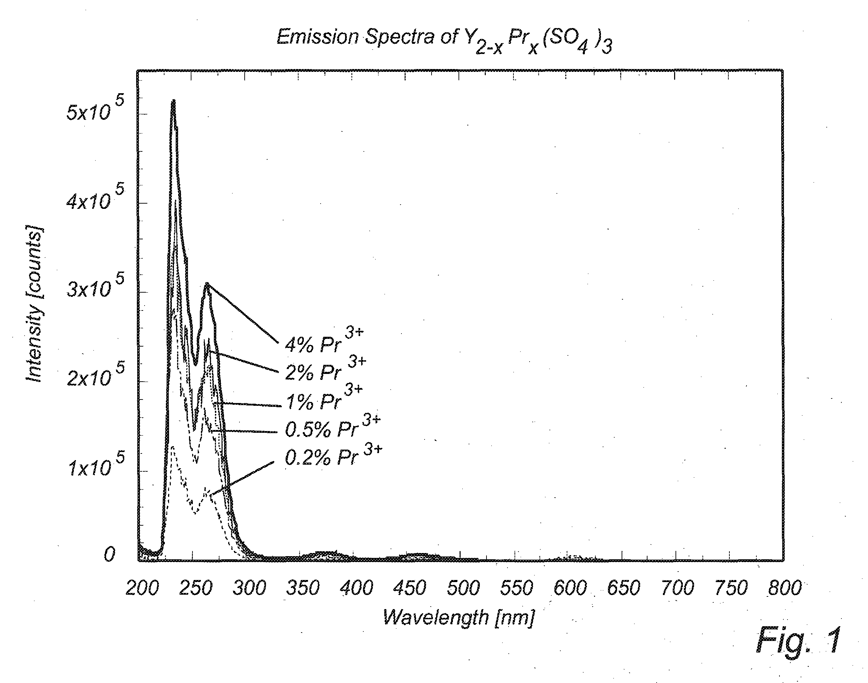

Wavelength converting compounds according to the invention have been found to have intense and efficient emission of UV radiation in the wavelength range of 200-300 nm, for example 200-280 nm or 220-300 nm. Fig. 1 shows the emission spectra of a wavelength converting compound according to embodiments of the invention having the formula Y₂(SO₄)₃:Pr (Pr³⁺ doped yttrium orthosulfate), at different concentrations of the dopant. The tested compounds contained Pr at a content of 0.2 atomic % (at.%), 0.5 at.%, 1 at.%, 2 at.% or 4 at.%, respectively. The dopant percentage refers to atomic %, i.e. the relative number of activator atoms (Me atoms) replacing atoms of the crystallographic site.

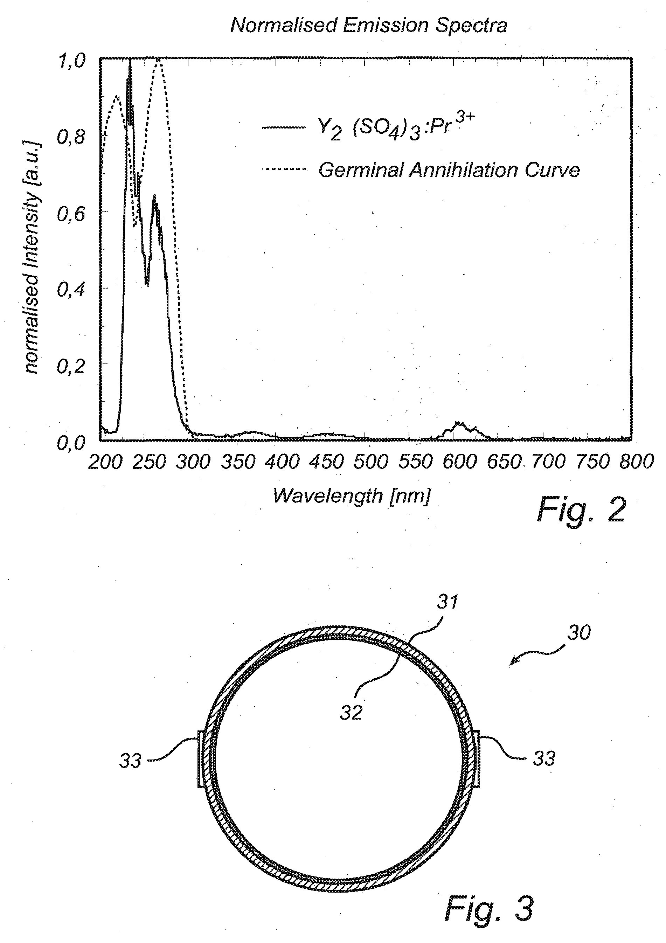

Advantageously, the emission spectrum of the wavelength converting compounds according to the invention have a large overlap with the germicidal action curve (GAC), which shows the germicidal effect of UV light on *E. coli.* This effect is mainly achieved by radiation of 200-300 nm. As can be seen in Fig. 2, the emission from a Pr³⁺ doped yttrium orthosulphate according to embodiments of the invention has a large spectral overlap with the germicidal action curve, the integral overlap being of about 71 %.

Because of its spectral characteristics, the wavelength converting compounds of the invention are useful as converters of very short wavelength UV radiation (typically VUV, having a wavelength of 100-200 nm) into UV light of 200-300 nm, for example 200-280 nm (representing part of the UV-C spectrum) or 220-300 nm, or 220-280 nm. The wavelength converting compounds of the invention may have strong absorption in the range of 100-200 nm, in particular 150-180 nm.

UV emission from the wavelength converting compounds according to embodiments of the invention may be useful in various applications. For example, due to large overlap with the germicidal action curve, the wavelength converting compounds may be particularly useful for ultraviolet germicidal irradiation to achieve sterilization, disinfection and/or purification, e.g. of food, air or water, such as drinking water, waste water, pool or pond water and the like, of objects such as laboratory or medical equipment, keyboards, personal care appliances such as tooth brushes and shavers, cosmetic tools, etc.

However, the wavelength converting compounds of the invention may also be useful for UV irradiation for purposes other than sterilization, disinfection and/or purification. For example, the UV emission of the present wavelength converting compounds may be used for medical or cosmetic treatment of humans or animals, e.g. cosmetic or medical treatment of the skin. Examples of cosmetic treatment by UV irradiation include tanning. Examples of medical treatment by UV irradiation include treatment of skin conditions and diseases, such as psoriasis, vitiligo, acne, and treatment of vitamin D deficiency.

Furthermore, UV irradiation with the emission wavelengths of the present wavelength converting compounds may be useful to achieve chemical reactions such as crosslinking, photopolymerization, photooxidation, photoreduction, and photocatalysis, and other photochemical applications.

Furthermore, UV irradiation with the emission wavelengths of the present wavelength converting compounds may be useful in the processing of semiconductor wafers, in particular for photoetching.

Hence, the wavelength converting compounds of the invention may be applied in UV emitting illumination devices for a wide range of applications. For example, a wavelength converting compound according to the invention may be applied in a UV emitting discharge lamp. Typically, the inner wall of the discharge vessel of a discharge lamp may be provided with a wavelength converting coating containing the wavelength converting compound.

A discharge lamp according to embodiments of the invention is shown in cross-section in Fig. 3. The discharge lamp 30 has the general structure of a conventional discharge lamp and comprises a cylindrical glass tube 31 forming a discharge vessel. The glass tube 31 contains a gas comprising one or more of Ar, Kr, Xe, F₂, Cl₂, Br₂, and I₂, typically Xe. The inner surface of the glass tube 31 is provided with a wavelength converting coating 32 comprising a wavelength converting material comprising a wavelength converting compound according to the invention. In embodiments of the invention, the coating 32 may comprise more than one type of wavelength converting compound. The discharge lamp 30 is also provided with conventional electrodes 33 for providing an electrical field across the vessel 31 and the gas contained therein.

Preferably, in embodiments of the invention, the discharge lamp is an excimer discharge lamp, such as a xenon (Xe) excimer discharge lamp, a neon (Ne) excimer discharge lamo, or a xenon/neon excimer discharge lamp.

A discharge lamp, in particular an Xe, Ne or Xe/Ne excimer discharge lamp may be applied in a medical device for phototherapy, in particular phototherapy of the skin; a cosmetic device for cosmetic treatment, in particular of the skins; a system for sterilization, disinfection and/or purification; a chemical reactor; and a system for processing, in particular photoetching, of semiconductor wafers.

The wavelength converting compounds of the present invention may be produced by reacting an oxide of Y, Lu, Sc La or Gd, respectively, with a sulphate or oxide of the trivalent cation intended as activator (in particular Bi³⁺, Pr³⁺ or Nd³⁺) in an acid medium, typically containing sulphuric acid. The reaction product is then disintegrated, e.g. by milling or grinding, and annealed at high temperature, for example at a temperature in the range of from 500°C to 900°C, to obtain the host lattice and to distribute the activator therein by diffusion. For example, said reacting can be achieved by dissolving said oxide of Y, Lu, Sc La or Gd, together with said sulphate or oxide in sulphuric acid, and heating the resulting solution to a temperature in the range of about 600°C to about 800°C for a time period of from 1 to 8 hours, such as from 2 to 6 hours, and typically about 4 hours, and removing said sulphuric acid medium e.g. by allowing it to evaporate.

### EXAMPLES

### Example I. Preparation and properties of Y₂(SO₄)₃:Pr(1%)

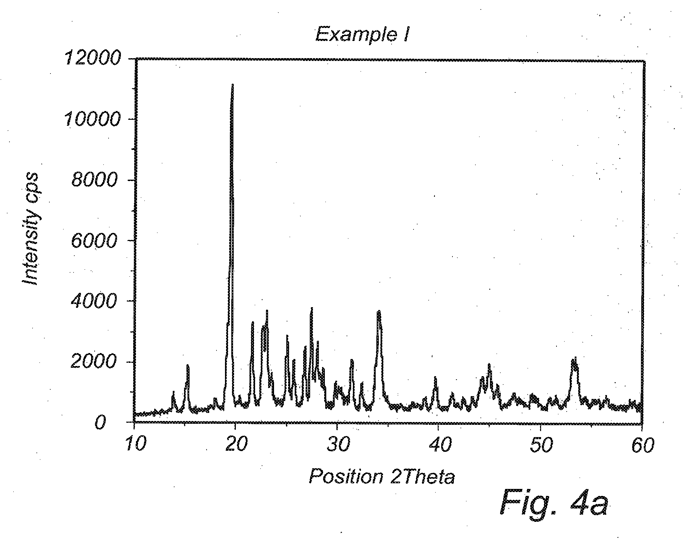

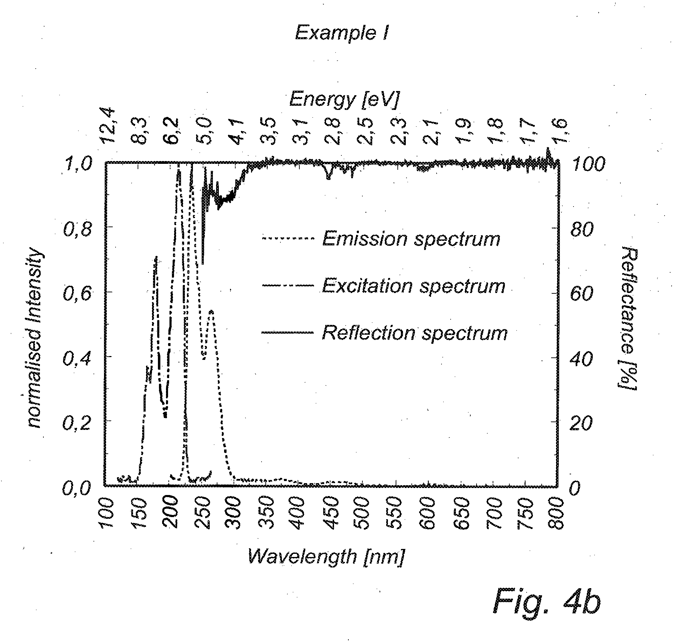

4.4936 g of Y₂O₃ and 0.06781 g of Pr₂(SO₄)₃*6H₂O as reagents were dissolved in 20 ml concentrated sulphuric acid and subsequently cooked until the acid was completely removed. The remaining powder was milled, filled into an alumina crucible, and annealed at 600 °C under nitrogen for 4 hours. The XRD pattern of the resulting compound, Y_{1.99}(SO₄):Pr_{0.01} (also written Y_{1.99}Pr_{0.01}(SO₄)₃) is shown in Fig. 4a. The reflection, excitation and emission spectra are shown in Fig. 4b. This wavelength converting compound has a quantum efficiency of >90 %.

### Example II. Preparation and properties of Lu₂(SO₄)₃:Pr(0.5%)

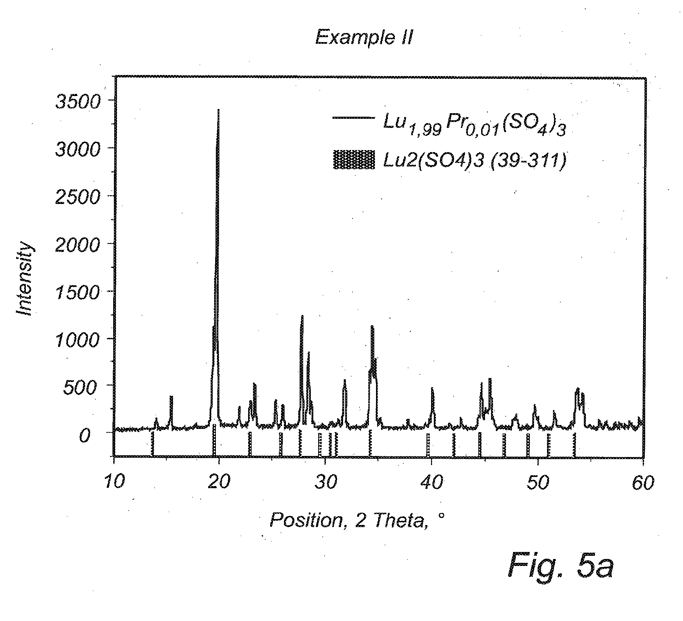

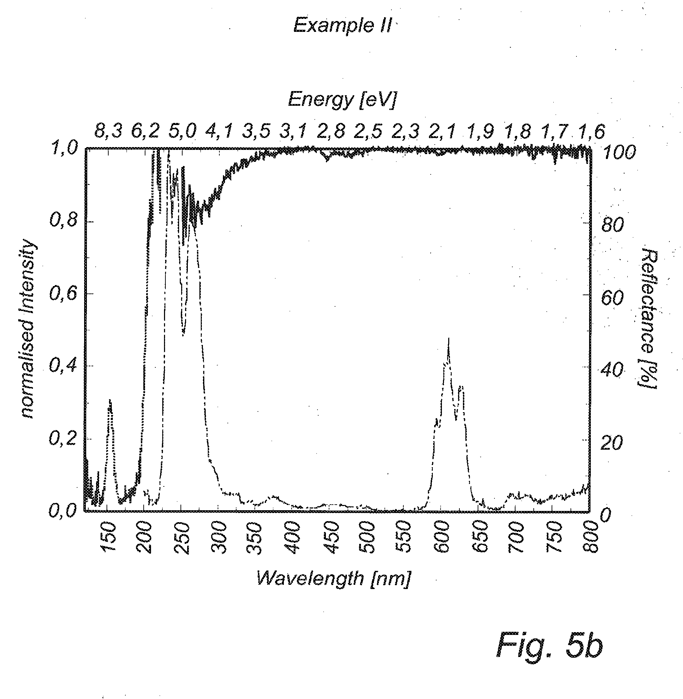

7.9188 g of Lu₂O₃ and 0.06781 g of Pr₂(SO₄)₃*6H₂O as reagents were dissolved in 20 ml concentrated sulphuric acid and subsequently cooked until the acid was completely removed. The remaining powder was milled, filled into an alumina crucible, and annealed at 800 °C under nitrogen for 4 hours. The XRD pattern of the resulting compound, Lu_{1.99}Pr_{0.01}(SO₄)₃, is shown in Fig. 5a. The reflection, excitation and emission spectra are shown in Fig. 5b.

### Example III. Preparation and properties of La₂(SO₄)₃:Pr(1%)

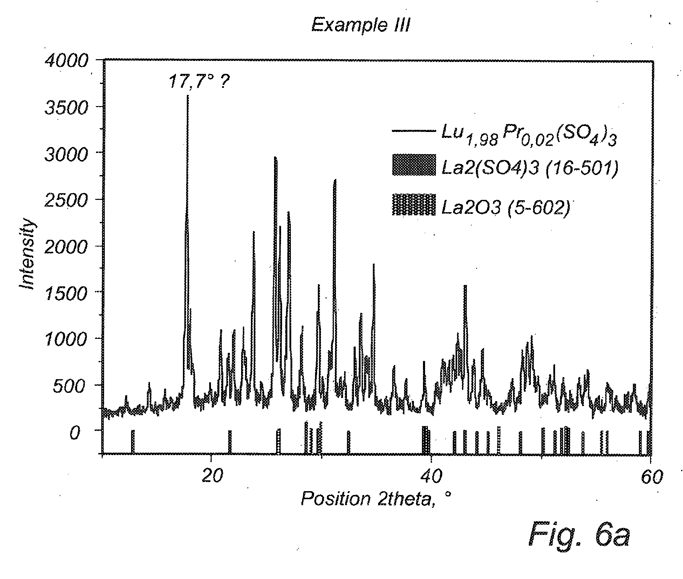

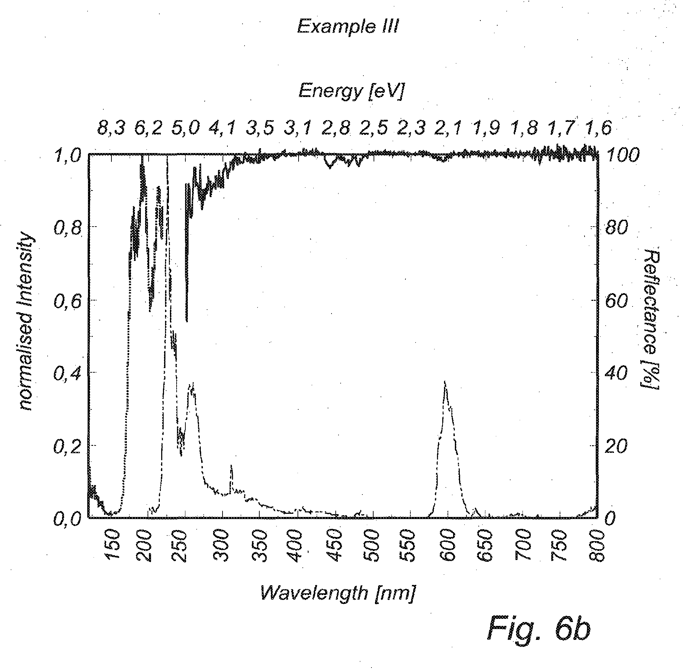

6.4836 g of La₂O₃ and 0.06781 g of Pr₂(SO₄)₃*6H₂O as reagents were dissolved in 20 ml concentrated sulphuric acid and subsequently cooked until the acid was completely removed. The remaining powder was milled, filled into an alumina crucible, and annealed at 800 °C under nitrogen for 4 hours. The XRD pattern of the resulting compound, La_{1.98}Pro_{0.02}(SO₄)₃, is shown in Fig. 6a. The reflection, excitation and emission spectra are shown in Fig. 6b.

### Example IV. Preparation and properties of Y₂(SO₄)₃:Nd(1%)

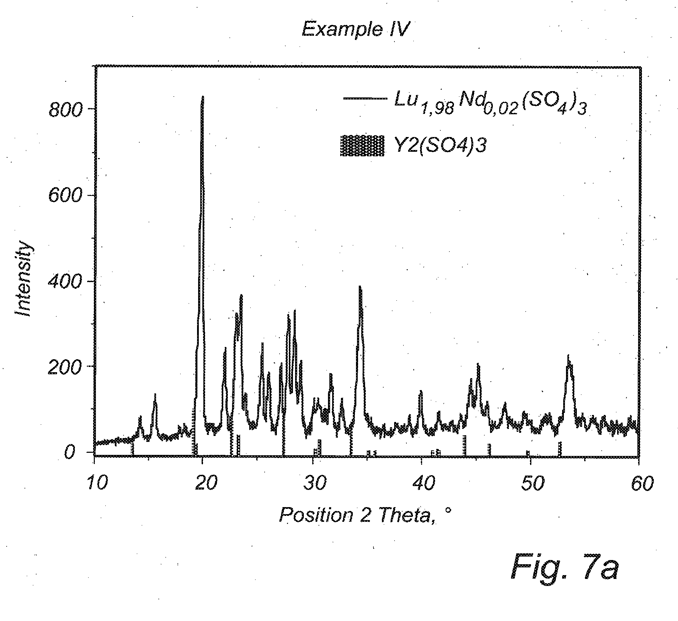

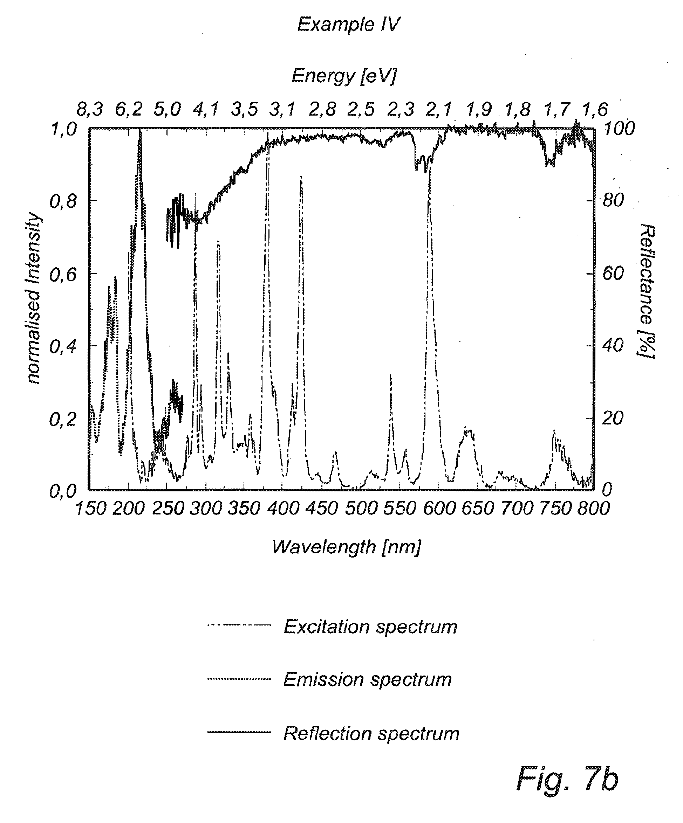

4.4710 g of Y₂O₃ and 0.06729 g of Nd₂O₃ as reagents were dissolved in 20 ml concentrated sulphuric acid and subsequently cooked until the acid was completely removed. The remaining powder was milled, filled into an alumina crucible, and annealed at 600 °C under nitrogen for 4 hours. The XRD pattern of the resulting compound, Y_{1.98}Nd_{0.02}(SO₄)₃, is shown in Fig. 7a. The reflection, excitation and emission spectra are shown in Fig. 7b.

### Example V. Preparation and properties of Lu₂(SO₄)₃:Bi(1%)

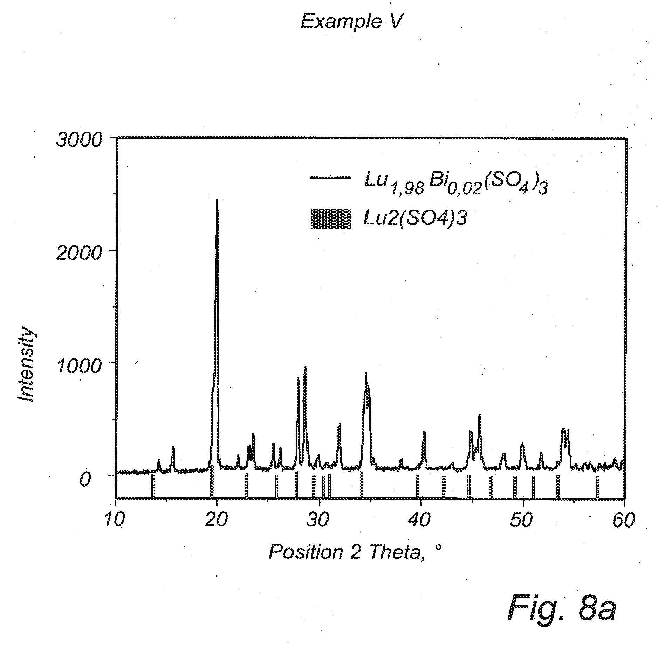

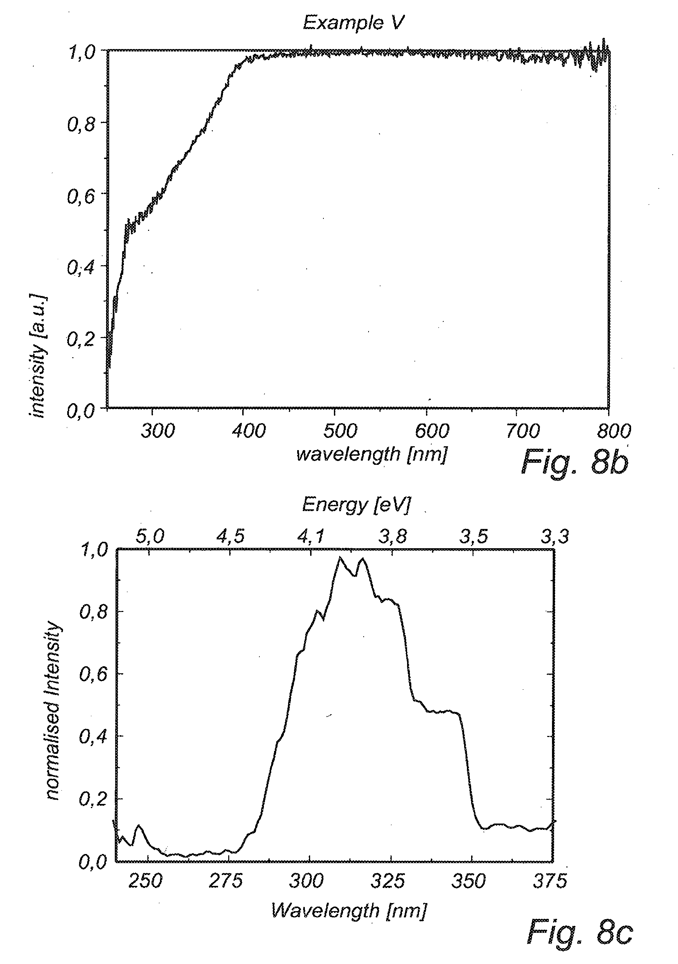

7.8991 g of Lu₂O₃ and 0.09319 g Bi₂O₃ as reagents were dissolved in 20 ml concentrated sulphuric acid and subsequently cooked until the acid was completely removed. The remaining powder was milled, filled into an alumina crucible, and annealed at 600 °C under nitrogen for 4 hours. The XRD pattern of the resulting compound, Lu_{1.98}Bi₀.₀₂(SO₄)₃, is shown in Fig. 8a. The reflection spectrum is shown in Fig. 8b, and the emission spectrum is shown in Fig 8c.

### Example VI. Preparation and properties of Lu₂(SO₄)₃:Nd(1%)

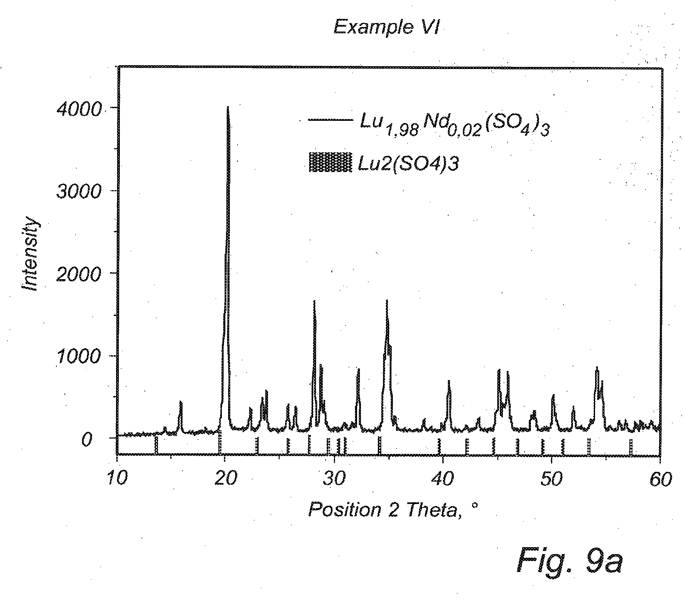

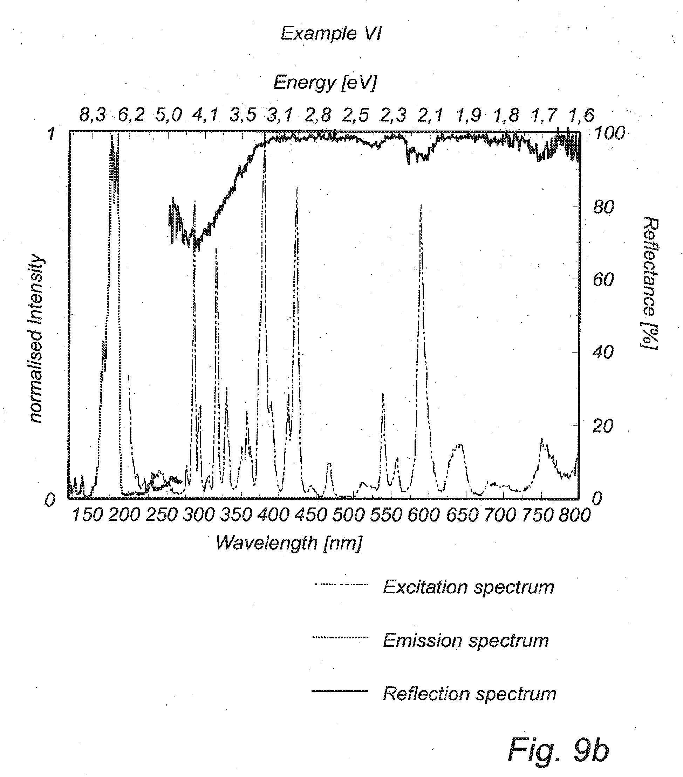

7.8991 g of Lu₂O₃ and 0.06729 g of Nd₂O₃ as reagents were dissolved in 20 ml concentrated sulphuric acid and subsequently cooked until the acid was completely removed. The remaining powder was milled, filled into an alumina crucible, and annealed at 600 °C under nitrogen for 4 hours. The XRD pattern of the resulting compound, Lu_{1.98}Nd_{0.02}(SO₄)₃, is shown in Fig. 9a. The reflection, excitation and emission spectrum is shown in Fig. 9b.

The person skilled in the art realizes that the present invention by no means is limited to the preferred embodiments described above. On the contrary, many modifications and variations are possible within the scope of the appended claims.

## Claims

1. A wavelength converting material comprising a wavelength converting compound of the formula (Y_{1-w-x-y-z}Sc_{w}LaₓGd_{y}Lu_{z})₂₋ₐ(SO₄)₃:Meₐ, wherein Me represents a trivalent cation or a mixture of trivalent cations capable of emitting UV-C radiation, and wherein each of w, x, y and z is in the range of from 0.0 to 1.0 and w+x+y+z ≤ 1.0, and wherein 0.0005 ≤ a ≤ 0.2.

2. A wavelength converting material according to claim 1, wherein Me comprises at least one of Pr³⁺, Nd³⁺ and Bi³⁺.

3. A wavelength converting material according to claim 1, wherein 0.002 ≤ a≤ 0.1.

4. A wavelength converting material according to claim 2, comprising additionally at least one further trivalent cation as a co-dopant.

5. A wavelength converting screen or coating, comprising a wavelength converting material according to claim 1.

6. An illumination device comprising a source of UV light to be converted, and a wavelength converting material according to claim 1 or a wavelength converting screen or coating according to claim 5 for converting UV light from said source.

7. An illumination device according to 6 which is a discharge lamp comprising a discharge vessel containing a gas comprising one or more of Ar, Kr, Xe, F₂, Cl₂, Br₂, and I₂, wherein at least part of a wall of the discharge vessel is provided with a wavelength converting material according to claim 1.

8. A medical device comprising a wavelength converting composition according to claim 1, a wavelength converting screen or coating according to claim 5, or an illumination device according to claim 6.

9. A medical device according to claim 8 which is a phototherapy device.

10. A cosmetic treatment device comprising a wavelength converting composition according to claim 1, a wavelength converting screen or coating according to claim 5, or an illumination device according to claim 6.

11. A system comprising an illumination device according to claim 6 for UV irradiation.

12. A system according to claim 11, which is a system for sterilization, disinfection or purification by germicidal UV irradiation.

13. Use of a wavelength converting composition according to claim 1 for sterilization, disinfection or purification.

14. Use of a wavelength converting composition according to claim 1 in a cosmetic treatment.

15. A method of producing a wavelength converting composition according to claim 1, comprising:
- reacting an oxide of Y, Lu, Sc La or Gd, with a sulphate or oxide of said trivalent cation in a sulphuric acid-containing medium; and
- removing said medium.

## Patentansprüche

1. Wellenlängenumwandlungsmaterial mit einer Wellenlängenumwandlungsverbindung der Formel (Y_{1-w-x-y-z}Sc_{w}LaₓGd_{y}Lu_{z})₂₋ₐ(SO₄)₃:Meₐ, wobei Me ein trivalentes Kation oder eine Mischung aus trivalenten Kationen darstellt, die imstande sind, UV-C-Strahlung zu emittieren, und wobei jedes von w, x, y, und z in dem Bereich von 0,0 bis 1,0 liegt und w+x+y+z ≤ 1,0, und wobei 0,0005 ≤ a ≤ 0,2.

2. Wellenlängenumwandlungsmaterial nach Anspruch 1, wobei Me mindestens einen von Pr³⁺, Nd³⁺ und Bi³⁺ umfasst.

3. Wellenlängenumwandlungsmaterial nach Anspruch 1, wobei 0,002 ≤ a ≤ 0,1.

4. Wellenlängenumwandlungsmaterial nach Anspruch 2 mit des Weiteren mindestens einem weiteren trivalenten Kation als ein Co-Dotierstoff.

5. Wellenlängenumwandlungsschirm oder -beschichtung mit einem Wellenlängenumwandlungsmaterial nach Anspruch 1.

6. Beleuchtungseinrichtung mit einer umzuwandelnden UV-Lichtquelle sowie einem Wellenlängenumwandlungsmaterial nach Anspruch 1 oder einem Wellenlängenumwandlungsschirm oder einer Wellenlängenumwandlungsbeschichtung nach Anspruch 5 zur Umwandlung von UV-Licht aus der Lichtquelle.

7. Beleuchtungseinrichtung nach Anspruch 6, bei der es sich um eine Entladungslampe mit einem Entladungsgefäß handelt, das ein Gas, bestehend aus einem oder mehreren von Ar, Kr, Xe, F₂, Cl₂, Br₂ und I₂, enthält, wobei zumindest eine Wand des Entladungsgefäßes mit einem Wellenlängenumwandlungsmaterial nach Anspruch 1 versehen ist.

8. Medizinische Vorrichtung mit einer Wellenlängenumwandlungszusammensetzung nach Anspruch 1, einem Wellenlängenumwandlungsschirm oder einer Wellenlängenumwandlungsbeschichtung nach Anspruch 5 oder einer Beleuchtungseinrichtung nach Anspruch 6.

9. Medizinische Vorrichtung nach Anspruch 8, bei der es sich um eine Lichttherapievorrichtung handelt.

10. Kosmetische Behandlungsvorrichtung mit einer Wellenlängenumwandlungszusammensetzung nach Anspruch 1, einem Wellenlängenumwandlungsschirm oder einer Wellenlängenumwandlungsbeschichtung nach Anspruch 5 oder einer Beleuchtungseinrichtung nach Anspruch 6.

11. System mit einer Beleuchtungseinrichtung nach Anspruch 6 zur UV-Bestrahlung.

12. System nach Anspruch 11, bei dem es sich um ein System zur Sterilisation, Desinfektion oder Reinigung durch keimtötende UV-Bestrahlung handelt.

13. Verwendung einer Wellenlängenumwandlungszusammensetzung nach Anspruch 1 zur Sterilisation, Desinfektion oder Reinigung.

14. Verwendung einer Wellenlängenumwandlungszusammensetzung nach Anspruch 1 in einer Kosmetikbehandlung.

15. Verfahren zur Erzeugung einer Wellenlängenumwandlungszusammensetzung nach Anspruch 1, wonach:
- ein Oxid von Y, Lu, Sc, La oder Gd mit einem Sulphat oder Oxid des trivalenten Kations in einem Schwefelsäure enthaltenden Medium zur Reaktion gebracht wird; und
- das Medium entfernt wird.

## Revendications

1. Matériau de conversion de longueur d'onde comprenant un composé de conversion de longueur d'onde de la formule (Y_{1-w-x-y-z}Sc_{w}LaₓGd_{y}Lu_{z})₂₋ₐ(SO₄)₃:Meₐ, dans lequel Me représente un cation trivalent ou un mélange de cations trivalents capable d'émettre un rayonnement UV-C, et dans lequel chacun de w, x, y et z est dans la plage de 0,0 à 1,0 et w+x+y+z ≤ 1,0, et dans lequel 0,0005 ≤ a ≤ 0,2.

2. Matériau de conversion de longueur d'onde selon la revendication 1, dans lequel Me comprend au moins un de Pr³⁺, Nd³⁺ et Bi³⁺.

3. Matériau de conversion de longueur d'onde selon la revendication 1, dans lequel 0,002 ≤ a ≤ 0,1.

4. Matériau de conversion de longueur d'onde selon la revendication 2, comprenant en outre au moins un cation trivalent supplémentaire en tant que co-dopant.

5. Ecran ou revêtement de conversion de longueur d'onde, comprenant un matériau de conversion de longueur d'onde selon la revendication 1.

6. Dispositif d'éclairage comprenant une source de lumière UV destinée à être convertie, et un matériau de conversion de longueur d'onde selon la revendication 1 ou un écran ou revêtement de conversion de longueur d'onde selon la revendication 5 pour convertir de la lumière UV à partir de ladite source.

7. Dispositif d'éclairage selon la revendication 6, qui est une lampe à décharge comprenant un récipient à décharge contenant un gaz comprenant un ou plusieurs de Ar, Kr, Xe, F₂, Cl₂, Br₂, et I₂, dans lequel au moins une partie d'une paroi du récipient à décharge est pourvue d'un matériau de conversion de longueur d'onde selon la revendication 1.

8. Dispositif médical comprenant une composition de conversion de longueur d'onde selon la revendication 1, un écran ou revêtement de conversion de longueur d'onde selon la revendication 5, ou un dispositif d'éclairage selon la revendication 6.

9. Dispositif médical selon la revendication 8, qui est un dispositif de photothérapie.

10. Dispositif de traitement cosmétique comprenant une composition de conversion de longueur d'onde selon la revendication 1, un écran ou revêtement de conversion de longueur d'onde selon la revendication 5, ou un dispositif d'éclairage selon la revendication 6.

11. Système comprenant un dispositif d'éclairage selon la revendication 6 pour irradiation UV.

12. Système selon la revendication 11, qui est un système pour la stérilisation, la désinfection ou la purification par irradiation UV germicide.

13. Utilisation d'une composition de conversion de longueur d'onde selon la revendication 1 pour la stérilisation, la désinfection ou la purification.

14. Utilisation d'une composition de conversion de longueur d'onde selon la revendication 1 dans un traitement cosmétique.

15. Procédé de production d'une composition de conversion de longueur d'onde selon la revendication 1, comprenant :
- la mise en réaction d'un oxyde de Y, Lu, Sc La ou Gd, avec un sulfate ou oxyde dudit cation trivalent dans un milieu contenant de l'acide sulfurique ; et
- l'élimination dudit milieu.
